# EUROPEAN PATENT SPECIFICATION

(11) **EP 2 862 497 B1**
(45) Date of publication and mention of the grant of the patent: **21.03.2018**
(21) Application number: 13804620.6
(22) Date of filing: 24.04.2013
(51) Int. Cl.: A61B 1/00, A61B 1/04, G02B 23/24

(54) **MANIPULATOR SYSTEM**
MANIPULATORSYSTEM
SYSTÈME DE MANIPULATEUR

(30) Priority: 14.06.2012 JP 2012134687
(43) Date of publication of application: 22.04.2015
(73) Proprietor: Olympus Corporation, Tokyo 192-8507 (JP)
(72) Inventor: HIROE, Atsushi, Hachioji-shi, Tokyo 192-8507 (JP); NARUSE, Masato, Hachioji-shi, Tokyo 192-8507 (JP)
(74) Representative: Gunzelmann, Rainer
(86) International application number: PCT/JP2013/062071
(87) International publication number: WO 2013/187136

(56) References cited:
- WO-A1-2011/060187
- JP-A- H08 224 248
- JP-A- 2003 230 073
- US-B1- 6 731 988

## Description

### {Technical Field}

The present invention relates to a manipulator system.

### {Background Art}

Conventionally, a monitoring system, which moves a display unit for displaying an endoscopic image when an endoscope is moved using supporting means for movably supporting the endoscope so that an optical axis at a distal end of the endoscope is arranged on an extension of a sight line looking in the display unit based on a movement amount of the supporting means, has been known (see PTL 1).

The monitoring system described in PTL 1 has an effect of obtaining a posture most suitable for surgery work without performing special work even when a user directly operates the endoscope to change an observation position in a body cavity of a patient.

### {Citation List}

### {Patent Literature}

### {PTL 1}

Japanese Unexamined Patent Application, Publication No. 2003-230073 US 6 731 988 B1 discloses a teleoperator system with telepresence including right and left hand controllers for control of right and left hand manipulators through use of a servomechanism that includes a computer. The teleoperator system further includes cameras which view the workspace from different angles for production of stereoscopic signal outputs.

Document JP H08 224248 A discloses a solution for setting the corresponding position relation of a master manipulator side and a slave manipulator on a treating side equally and for improving operability by applying such control to detect the angle of an angle detecting means of each shaft of a master arm for movement for moving a manipulator correspondingly.

### {Summary of Invention}

### {Technical Problem}

However, an instrument projecting from a distal end surface of an endoscope is operated when a user operates an operation handle with both his/her hands, and a bending portion provided at a distal end of the endoscope is generally operated by a switch without the user greatly moving his/her hands and feet. When the degree of bending of the bending portion is changed to change a direction of view of the endoscope, a direction of the instrument displayed on a display unit changes even if the instrument is not operated.

That is, movement directions of the operation handle when the instrument is made to perform the same operation are the same before and after an operation of the bending portion, while the movement direction of the instrument displayed on the display unit changes. Thus, the user needs to operate the operation handle while always confirming which are forward and backward and rightward and leftward directions of the instrument in the display unit in his/her head, and cannot perform an intuitive operation.

The present invention has been made in view of the aforementioned circumstances, and is directed to providing a manipulator system capable of intuitively operating an instrument displayed on a display unit even if an observation direction of a subject is switched.

### {Solution to Problem}

To attain the aforementioned object, the present invention provides the solution according to claim 1 and the further advantageous embodiments as defined in the dependent claims. There is provided a manipulator system including a treatment unit that treats a subject, an imaging unit that images the subject and the treatment unit, a display unit that displays an image captured by the imaging unit, a display unit driving unit that supports and moves the display unit, an operation unit that operates the treatment unit, an operation unit driving unit that supports and moves the operation unit, and a control unit that controls the display unit driving unit and the operation unit driving unit to bring a relative positional relationship between the display unit and the operation unit closer to a relative positional relationship between the imaging unit and the treatment unit.

When the imaging unit is moved relative to the subject and the treatment unit, to change a direction in which the subject and the treatment unit are imaged, the relative positional relationship between the imaging unit and the treatment unit changes. Thus, the control unit performs control to cause the display unit driving unit to move the display unit while causing the operation unit driving unit to move the operation unit. The position of the imaging unit relative to the treatment unit changes so that an image obtained by capturing the subject and the treatment unit from a different angle is displayed on the display unit.

In this case, the control unit controls the display unit driving unit and the operation unit driving unit to bring the relative positional relationship between the display unit and the operation unit approximate to the relative positional relationship between the imaging unit and the treatment unit. Thus, a relative relationship of a movement direction of the operation unit to a movement direction of the treatment unit in the image displayed on the display unit can be inhibited from greatly changing before and after the change in the position of the imaging unit. The user can continue to intuitively operate the treatment unit displayed on the display unit.

In the aforementioned aspect, the manipulator system may include a detection unit that detects an imaging unit angle of the subject by the imaging unit, in which the control unit may control the display unit driving unit and the operation unit driving unit based on the imaging unit angle detected by the detection unit.

Thus, the control unit can control the display unit driving unit and the operation unit driving unit to bring the relative positional relationship between the display unit and the operation unit closer to the relative positional relationship between the imaging unit and the treatment unit based on the imaging unit angle of the subject by the imaging unit detected by the detection unit.

In the aforementioned aspect, the display unit driving unit may change a display unit angle serving as an angle between a normal to the display unit and a horizontal direction.

Thus, the imaging unit can change, when it changes the imaging unit angle of the subject, the display unit angle to match this. Thus, the user who is viewing the display unit can feel as if the observation direction of the subject changed depending on a change in his/her field of view, and can more intuitively perform treatment for the subject.

In the aforementioned aspect, the operation unit driving unit may change an operation unit angle serving as an angle between a straight line connecting the operation unit and the display unit and a horizontal direction.

Thus, the imaging unit can change, when it changes the imaging unit angle of the subject, the operation unit angle to match this. Thus, the operation unit for operating the treatment unit moves together with the treatment unit that has moved on the image displayed on the display unit. Therefore, the user who is viewing the display unit can more intuitively determine a movement direction of the operation unit for moving the treatment unit in a desired direction before and after the change in the observation direction of the subject.

In the aforementioned aspect, the control unit may control the display unit driving unit so that the imaging unit angle and the display unit angle match each other.

In the aforementioned aspect, the control unit may control the operation unit driving unit so that the imaging unit angle and the operation unit angle match each other.

In the aforementioned aspect, the operation unit driving unit may change an operation unit angle serving as an angle between a straight line connecting the operation unit and the display unit and a horizontal direction, and the control unit may control the display unit driving unit and the operation unit driving unit so that the imaging unit angle, the operation unit angle, and the display unit angle match one another.

Thus, the imaging unit can change, when it changes the imaging unit angle of the subject, the display unit angle and the operation unit angle to match this.

In the aforementioned aspect, the manipulator system may further include a function storage unit that stores a first function representing a relationship between the imaging unit angle and the display unit angle, in which the control unit may control the display unit driving unit based on the first function stored in the function storage unit.

Thus, the display unit angle can simply be determined using the first function stored in the function storage unit so that treatment can be performed at high speed.

In the aforementioned aspect, the first function may be a function having a slope decreasing toward both ends of an angular range of the imaging unit angle.

Thus, when the display unit angle is changed according to the first function depending on the change in the imaging unit angle, the display unit angle also greatly changes as the imaging unit angle greatly changes to come closer to both ends of the angular range thereof. However, the slope of the first function is decreased as the imaging unit angle comes closer to both the ends of the angular range so that the user may not be forced to have an unrelaxed posture.

In the aforementioned aspect, the first function may be a function in which a change amount of the display unit angle relative to a change amount of the imaging unit angle is one or less over an entire angular range of the imaging unit angle.

Thus, when the display unit angle is changed according to the first function depending on the change in the imaging unit angle, the display unit angle greatly changes as the imaging unit angle greatly changes to come closer to both ends of the angular range thereof. However, the slope of the first function is set to one or less over the entire angular range of the imaging unit angle so that the user may not be forced to have an unrelaxed posture at both the ends of the angular range.

In the aforementioned aspect, the manipulator system may further include a function storage unit that stores a second function representing a relationship between the imaging unit angle and the operation unit angle, in which the control unit may control the operation unit driving unit based on the second function stored in the function storage unit.

Thus, the operation unit angle can simply be determined using the second function stored in the function storage unit so that treatment can be performed at high speed.

In the aforementioned aspect, the second function may be a function having a slope decreasing toward both ends of an angular range of the imaging unit angle.

Thus, when the operation unit angle is changed according to the second function depending on the change in the imaging unit angle, the operation unit angle greatly changes as the imaging unit angle greatly changes to come closer to both the ends of the angular range thereof. However, the slope of the second function is decreased as the imaging unit angle comes closer to both the ends of the angular range so that the user may not be forced to have an unrelaxed posture.

In the aforementioned aspect, the second function may be a function in which a change amount of the operation unit angle relative to a change amount of the imaging unit angle is one or less over an entire angular range of the imaging unit angle.

Thus, when the operation unit angle is changed according to the second function depending on the change in the imaging unit angle, the operation unit angle also greatly changes as the imaging unit angle greatly changes to come closer to both the ends of the angular range thereof. However, the slope of the second function is set to one or less over the entire angular range of the imaging unit angle so that the user may not be forced to have an unrelaxed posture at both the ends of the angular range.

### {Advantageous Effects of Invention}

The present invention produces an effect of intuitively operating an operation unit displayed on a display unit even if an observation direction of a subject is switched.

### {Brief Description of Drawings}

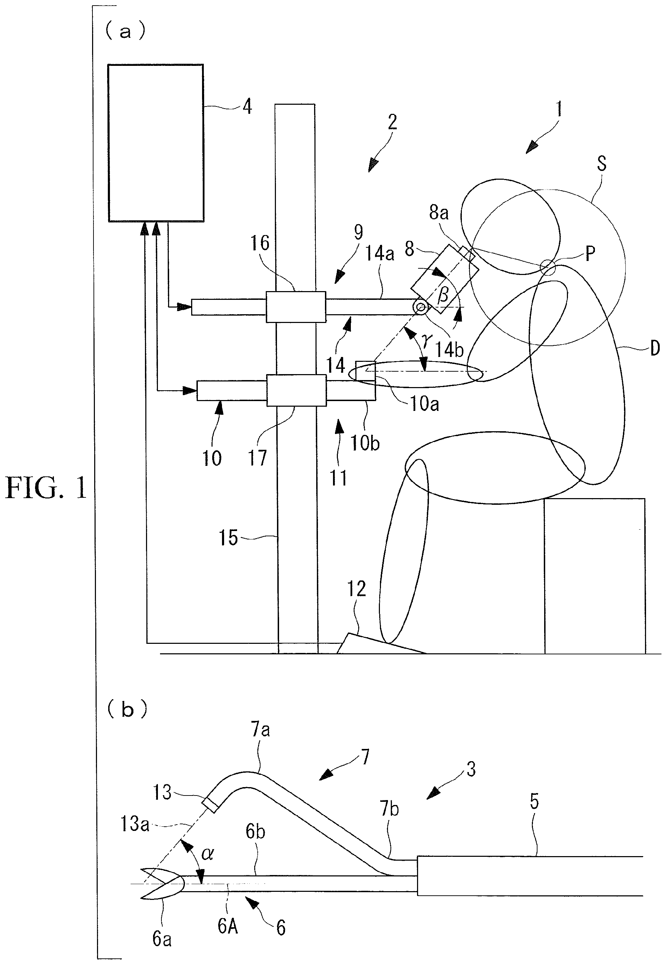

{Fig. 1}
   Figs. 1 (a) and 1 (b) are diagrams respectively illustrating a master device and a slave device in a manipulator system according to an embodiment of the present invention.
{Fig. 2}
   Fig. 2 is a block diagram illustrating an example of a controller provided in the manipulator system illustrated in Fig. 1.
{Fig. 3}
   Fig. 3 is a graph illustrating a relationship between an angle α and angles β and γ provided in the controller illustrated in Fig. 1.
{Fig. 4}
   Figs. 4 (a) and 4 (b) are diagrams respectively illustrating a master device and a slave device in a state where an observation position of the manipulator system illustrated in Fig. 1 has been changed.
{Fig. 5}
   Fig. 5 is a diagram illustrating an example of an image to be displayed on a monitor of the manipulator system illustrated in Fig. 1 and obtained when an affected area and an instrument are observed at a shallower angle.
{Fig. 6}
   Fig. 6 is a diagram illustrating an image obtained when the affected area and the instrument are observed at a deeper angle than that in the state illustrated in Fig. 4.
{Fig. 7}
   Fig. 7 is a block diagram illustrating a modification of a controller in the manipulator system illustrated in Fig. 1.
{Fig. 8}
   Fig. 8 is a diagram illustrating a modification of first and second functions provided in the controller in the manipulator system illustrated in Fig. 1.
{Fig. 9}
   Fig. 9 is a block diagram illustrating a controller including the first function illustrated in Fig. 7 and the second function illustrated in Fig. 8.
{Fig. 10}
   Fig. 10 is a graph illustrating a modification of the first function illustrated in Fig. 7 and the second function illustrated in Fig. 8.
{Fig. 11}
   Fig. 11 is a graph illustrating another modification of the first function illustrated in Fig. 7 and the second function illustrated in Fig. 8.
{Fig. 12}
   Fig. 12 is a block diagram illustrating another modification of the controller illustrated in Fig. 2.

### {Description of Embodiments}

A manipulator system 1 according to an embodiment of the present invention will be described below with reference to the drawings.

The manipulator system 1 according to the present embodiment is a medical manipulator system 1, and includes a master device 2 illustrated in Fig. 1 (a), a slave device 3 illustrated in Fig. 1 (b), and a controller (control unit) 4 that controls the devices.

The slave device 3 includes a cylindrical mantle tube 5 composed of a rigid material to be inserted into the body of a patient, an instrument (treatment unit) 6 that projects from an opening at a distal end of the mantle tube 5 to treat an affected area (subject) A in the body of the patient, and an endoscope 7 that projects from the opening at the distal end of the mantle tube 5 to capture the affected area A and the instrument 6.

The master device 2 includes a monitor (display unit) 8 that displays an image captured by the endoscope 7, a monitor driving unit 9 that moves the monitor 8, a handle (operation unit) 10 that operates the instrument 6, a handle driving unit 11 that moves the handle 10, and a foot switch 12.

The instrument 6 includes forceps 6a that treats the affected area A and an instrument arm 6b, which has one or more joints (not illustrated) having the forceps 6a attached to its distal end. The instrument arm 6b elongates and contracts the joints to enable the forceps 6a at the distal end to advance and retreat in a longitudinal direction of the mantle tube 5. The instrument 6 includes a pair of right and left instruments 6 (only one of them is displayed in the figure) provided to be respectively operated with both hands of a doctor D.

Motors (not illustrated) or the like are respectively provided in the instrument arm 6b and the forceps 6a, and are controlled by the controller 4.

The endoscope 7 has an illumination unit (not illustrated) and a camera (imaging unit) 13 arranged at its distal end, and includes two bending portions 7a and 7b spaced apart from each other in the longitudinal direction on the side of a proximal end of the camera 13. The two bending portions 7a and 7b are configured to be bent within a plane perpendicular to a plane (a horizontal plane in the present embodiment) 6A in which the two instruments 6 are arranged, as illustrated in Fig. 1 (b), and can make an optical axis 13a of the camera 13 intersect the aforementioned plane 6A and adjust an intersection angle α therebetween.

The endoscope 7 incudes a motor (not illustrated) for bending the bending portions 7a and 7b in the endoscope 7 and a motor (not illustrated) for making the bending portions 7a and 7b advance and retreat to and from the mantle tube 5 in the longitudinal direction thereof. Each of the motors includes an encoder (detection unit) that detects a bending angle of each of the bending portions 7a and 7b and its movement distance along the longitudinal direction of the mantle tube 5. The controller 4 controls each of the motors. The monitor 8 includes a display (not illustrated) that displays an image captured by the camera 13 in the endoscope 7 and an eyepiece portion 8a.

The monitor driving unit 9 includes a monitor arm 14 that supports the monitor 8, and a linear actuator 16 that moves the monitor arm 14 in a horizontal direction and a vertical direction relative to a support frame 15 fixed to a ground surface. The monitor arm 14 includes a base portion 14a that swingably supports the monitor 8 around a horizontal axis, and a swing actuator 14b that swings the monitor 8 relative to the base portion 14a.

The handle 10 includes a pair of right and left handles 10 provided to be operated with both the hands of the doctor D, and includes a grip 10a independently held in both the hands of the doctor D and an operation arm 10b, having one or more joints, which makes a position of the grip 10a advance and retreat in a horizontal direction.

The grip 10a includes a grip detection portion (not illustrated) that detects the degree of grip, and the operation arm 10b includes a position detection portion (not illustrated) that detects a position in the horizontal direction of the grip 10a.

The handle driving unit 11 includes a linear actuator 17 that moves the operation arm 10b in a horizontal direction and a vertical direction relative to the support frame 15.

The foot switch 12 is foot-operated by the doctor D, and its stepping amount can be adjusted.

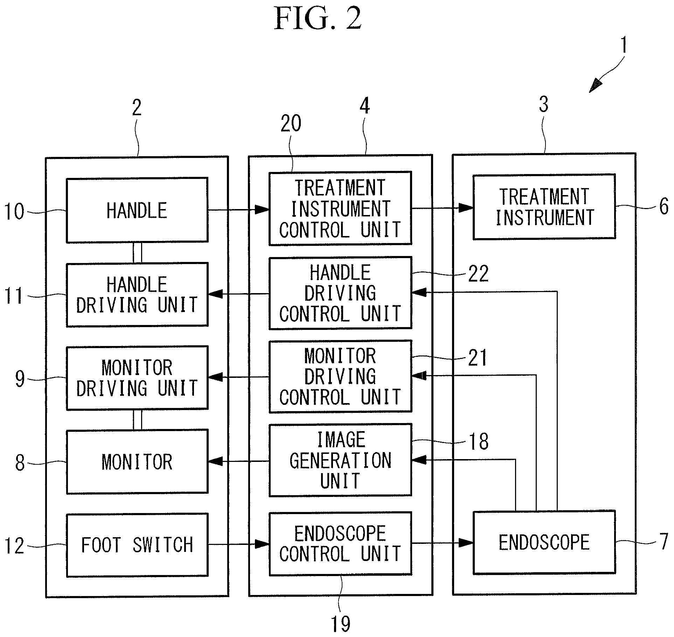

The controller 4 includes an image generation unit 18 that generates an image based on an image signal captured by the camera 13 in the endoscope 7 and outputs the generated image to the monitor 8, as illustrated in Fig. 2.

The controller 4 includes an endoscope control unit 19 that calculates an operation amount of each of the motors in the endoscope 7 based on the stepping amount of the foot switch 12, and instructs each of the motors. The endoscope control unit 19 advances the endoscope 7 in the longitudinal direction while increasing the bending angle of each of the bending portions 7a and 7b when the doctor D steps the foot switch 12, for example. Thus, while the optical axis 13a of the camera 13 is made to intersect the affected area, the angle α to the horizontal plane 6A is increased.

The controller 4 includes an instrument control unit 20 that calculates an operation amount of each of the motors in the instrument arm 6b and the forceps 6a based on an operation amount of the handle 10 by the doctor D, and instructs each of the motors.

The instrument control unit 20 controls the motor in the forceps 6a so that the forceps 6a are closed and opened, respectively, when the doctor D holds the grip 10a in the handle 10 and when the doctor D releases the grip 10a, for example.

The instrument control unit 20 calculates, when the doctor D advances the handle 10 in the horizontal direction, a movement amount by which a position of the forceps 6a is advanced based on a movement amount in the horizontal direction detected by the position detection portion, elongates the joints of the instrument arm 6b, and advances the position of the forceps 6a.

The controller 4 includes a monitor driving control unit 21 that controls the monitor driving unit 9 and a handle driving control unit 22 that controls the handle driving unit 11 when the bending angles of the two bending portions 7a and 7b, which have been detected by the encoder provided in the motor in the endoscope 7, are input thereto.

The monitor driving control unit 21 calculates the angle α between the optical axis 13a of the camera 13 and the horizontal plane 6A based on the bending angles of the two bending portions 7a and 7b, and controls the monitor driving unit 9 so that an angle β between an optical axis of the eyepiece portion 8a in the monitor 8 and a horizontal plane matches the angle α of the optical axis 13a of the camera 13.

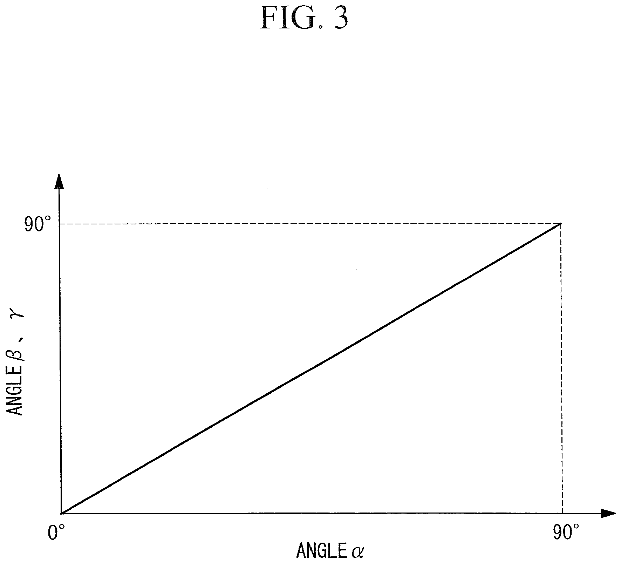

More specifically, in the present embodiment, the monitor driving control unit 21 controls the monitor driving unit 9 so that the angle α and the angle β change based on a function (first function) that changes in a directly proportional relationship as illustrated in Fig. 3.

The monitor driving control unit 21 not only performs control so that the angle β between the monitor 8 and the base portion 14a in the monitor arm 14 matches the angle α of the optical axis 13a of the camera 13 by operating the swing actuator 14b but also adjusts positions in the horizontal direction and the vertical direction of the monitor arm 14 relative to the support frame 15 by operating the linear actuator 16.

More specifically, in the present embodiment, the monitor driving control unit 21 stores positional coordinates of a rotation center P of its head portion arranged at a position of the neck of the doctor D, as illustrated in Fig. 1 (a). The monitor driving control unit 21 operates the swing actuator 14b and the linear actuator 16 so that the eyepiece portion 8a in the monitor 8 moves along the circle S within a vertical plane centered around the rotation center P.

The handle driving control unit 22 controls the handle driving unit 11 so that a relative positional relationship between the eyepiece portion 8a in the monitor 8 and the grip 10a matches a relative positional relationship between the camera 13 and the instrument 6. Matching between the relative positional relationships means that the angle α between the optical axis 13a of the camera 13 and the horizontal plane 6A substantially matches an angle γ between a straight line connecting the eyepiece portion 8a and the grip 10a and a horizontal plane. The handle driving unit 11 is controlled so that the angles α and γ change based on a function (second function) that changes in a directly proportional relationship as illustrated in Fig. 3.

In the present embodiment, the handle driving control unit 22 controls the handle driving unit 11 so that the angles α and γ match each other and a ratio of a distance between the camera 13 and the instrument 6 to a distance between the eyepiece portion 8a and the grip 10a becomes constant (e.g., 1 : 10).

Thus, the grip 10a is arranged on an extension of the optical axis of the eyepiece portion 8a in the monitor 8 inclined at the angle β that matches the angle α between the optical axis 13a of the camera 13 and the horizontal plane 6A, as illustrated in Fig. 1 (a).

The action of the manipulator system 1 according to the present embodiment thus configured will be described below.

When the doctor D treats the affected area A of the patient using the manipulator system 1 according to the present embodiment, the doctor D arranges the endoscope 7 and the instrument 6 in the slave device 3 in proximity to the affected area A in the body of the patient, and capture the affected area A and the instrument 6 using the camera 13 in the endoscope 7.

Image information captured by the endoscope 7 is fed to the image generation unit 18 in the controller 4, and an image to be displayed on the monitor 8 is generated in the image generation unit 18. The generated image is fed to the monitor 8 in the master device 2, and is displayed on the display provided in the monitor 8.

The doctor D holds the grip 10a in the master device 2, puts his/her foot on the foot switch 12 while applying his/her eye to the eyepiece portion 8a in the monitor 8 to observe the image displayed on the display. The monitor driving control unit 21 controls the monitor driving unit 9 so that the angle α of the optical axis 13a of the camera 13 and the angle β of the monitor 8 match each other and the eyepiece portion 8a is arranged on the circle S passing through the eye of the doctor D centered around the rotation center P, at the neck of the doctor D, which has previously been stored. Thus, the doctor D can observe the image displayed on the display in a natural posture upon applying the eye to the eyepiece portion 8a.

The handle driving control unit 22 controls the handle driving unit 11 so that the angle γ between the straight line connecting the eyepiece portion 8a and the grip 10a and the horizontal plane matches the angle α between the optical axis 13a of the camera 13 and the horizontal plane 6A. Thus, the doctor D who is viewing the image via the eyepiece portion 8a can recognize the image as if the grip 10a held with both his/her hands were the same as the instrument 6 on the image.

That is, the degree of grip of the grip 10a that the doctor D holds with both his/her hands is changed so that the instrument 6 within the image can be opened and closed in synchronization with the change. When the grip 10a is pushed and pulled in a direction matching a forward and backward direction of the instrument 6 on the image, the grip 10a moves in the forward and backward direction, and the instrument 6 within the image can be moved back and forth in synchronization with the movement.

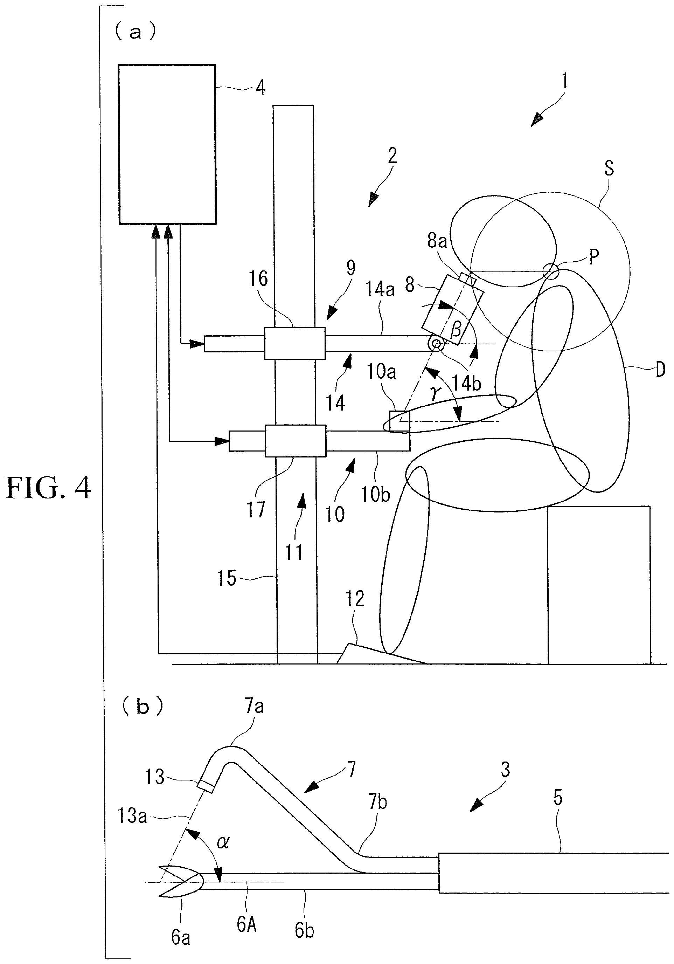

The doctor D operates the foot switch 12 with his/her foot when he/she desires to change an observation angle of the affected area A and the instrument 6. Thus, the stepping amount of the foot switch 12 is transmitted to the endoscope control unit 19 in the controller 4, and the endoscope control unit 12 changes the bending angles of the two bending portions 7a and 7b in the endoscope 7 depending on the stepping amount. When the stepping amount of the foot switch 12 is changed from the state illustrated in Fig. 1 (b), for example, the bending angles of the two bending portions 7a and 7b change so that the angle α between the optical axis 13a of the camera 13 and the horizontal plane 6A increases, as illustrated in Fig. 4 (b).

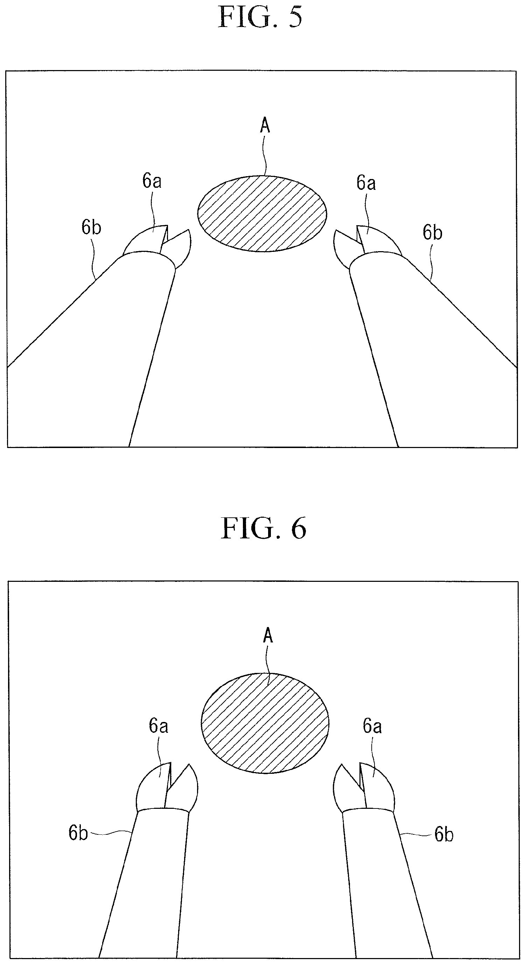

As a result, the image displayed on the display in the monitor 8 changes from a state illustrated in Fig. 5 to a state illustrated in Fig. 6.

When the bending angles of the bending portions 7a and 7b in the endoscope 7 change, an angle detected by the encoder in the motor for driving the bending portions 7a and 7b is fed to the monitor driving control unit 21 and the handle driving control unit 22.

The monitor driving control unit 21 calculates the angle α between the optical axis 13a of the camera 13 in the endoscope 7 and the horizontal plane 6A from the angle of each of the bending portions 7a and 7b, which has been detected by the encoder, and operates the swing actuator 14b so that the swing angle β of the monitor 8 matches the angle α based on the angle α. The doctor D can be made to feel as if an observation direction of the affected area A and the instrument 6 were changed by changing a direction of his/her own sight line by changing the swing angle β of the monitor 8, as illustrated in Fig. 4 (a), to match changes in an observation direction of the affected area A and the like in the image, and can perform a more intuitive operation of the instrument 6.

In this case, the monitor driving control unit 21 operates the swing actuator 14b and the linear actuator 14a in synchronization with each other so that the eyepiece portion 8a in the monitor 8 moves along the circle S centered around the center position P at which the neck of the doctor D is bent, and stops operating both the actuators 14a and 14b at the time point where the angle β matches the angle α. Thus, the doctor D can always observe the image displayed on the display from the eyepiece portion 8a in a relaxed posture only by changing inclination of the neck without moving other parts of the body.

Further, according to the present embodiment, the handle driving control unit 22 calculates the angle α between the optical axis 13a of the camera 13 in the endoscope 7 and the horizontal plane 6A from the angle of each of the bending portions 7a and 7b, which have been detected by the encoder, and controls the handle driving unit 11 based on the angle α. That is, the handle driving control unit 22 controls the handle driving unit 11 so that the angle γ between the straight line connecting the eyepiece portion 8a and the grip 10a and the horizontal plane matches the angle α between the optical axis 13a of the camera 13 and the horizontal plane 6A, as illustrated in Fig. 4 (a). Thus, the doctor D who is viewing the image via the eyepiece portion 8a can recognize the image as if the instrument 6 on the image were always at a position of the grip 10a.

More specifically, for the doctor D who is viewing the image displayed on the display via the eyepiece portion 8a, a movement direction of the instrument 6 is a direction intersecting a display screen in the state illustrated in Fig. 5, but changed to a direction more along the display screen in the state illustrated in Fig. 6. The doctor D can cause the instrument 6 to advance and retreat by moving the grip 10a in a direction matching a movement direction of the instrument 6 on the image. Therefore, the instrument 6 can be driven by an intuitive operation of the grip 10a so that the affected area A can more easily be treated.

While a rigid straight tubular member is illustrated as the mantle tube 5 in the present embodiment, the present invention is not limited to this. A mantle tube having a bending portion (not illustrated) at a halfway position in a longitudinal direction may be used to integrally change directions of the instrument 6 and the endoscope 7. A bending direction of the bending portion of the mantle tube 5 may be any of a horizontal direction, a vertical direction, and their combination.

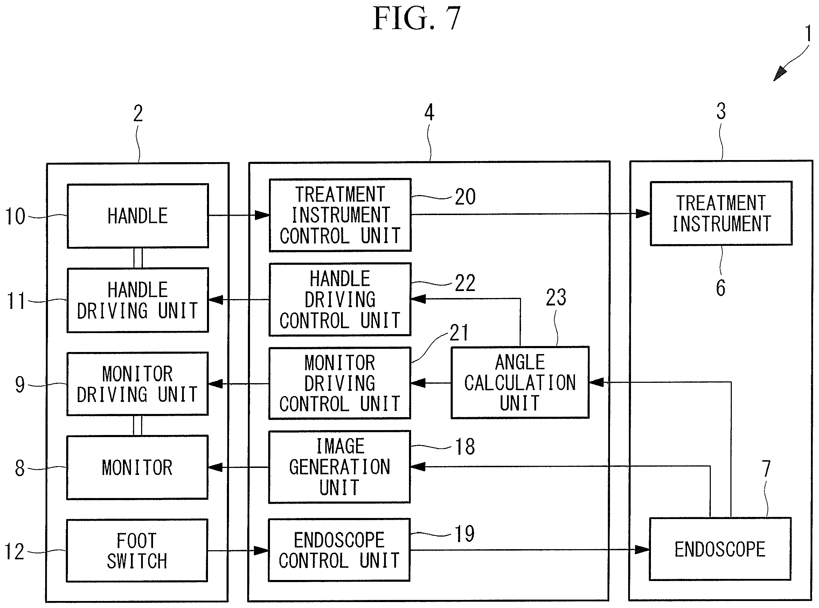

While the monitor driving control unit 21 and the handle driving control unit 22 respectively calculate the angle α between the optical axis 13a of the camera 13 and the horizontal plane 6A based on a detection signal by the encoder included in the motor for bending the bending portions 7a and 7b in the endoscope 7 in the present embodiment, a common angle calculation unit 23 may be arranged, as illustrated in Fig. 7, instead thereof to input the angle α calculated by the angle calculation unit 23 to the monitor driving control unit 21 and the handle driving control unit 22.

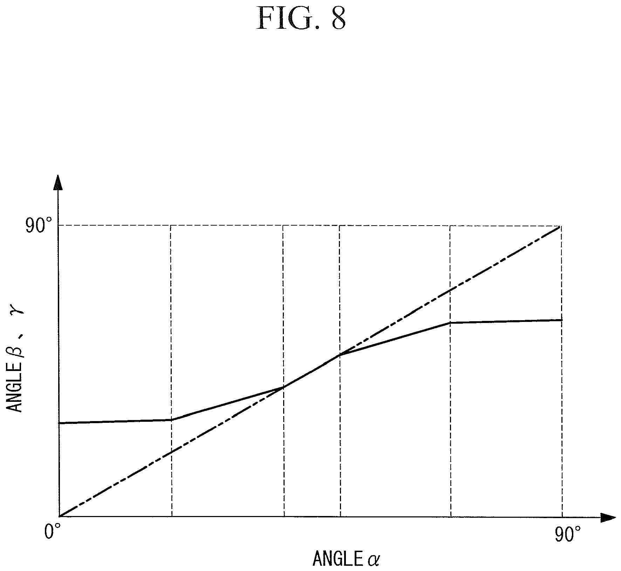

While the angle β of the monitor 8 and the angle γ of the straight line connecting the eyepiece portion 8a and the grip 10a are adjusted based on the first function and the second function serving as directly proportional functions with a proportionality coefficient 1, first and second functions illustrated in Fig. 8 may be used instead thereof.

An angular range (0° to 90°) of the angle α of the optical axis 13a of the camera 13 is divided into five areas. The first and second functions illustrated in Fig. 8 are respectively represented as directly proportional functions with a proportionality coefficient 1 in which the angles β and γ are proportional to the angle α in a central area (40° to 50°), have their slopes gradually decreasing toward both ends of the angular range in four areas (0° to 20°, 20° to 40°, 50° to 70°, and 70° to 90°) on both sides thereof, and are discontinuous functions as the entire angular range. In Fig. 8, a dot and dash line indicates the function, as illustrated in Fig. 3, which is a directly proportional function with a proportionality coefficient 1.

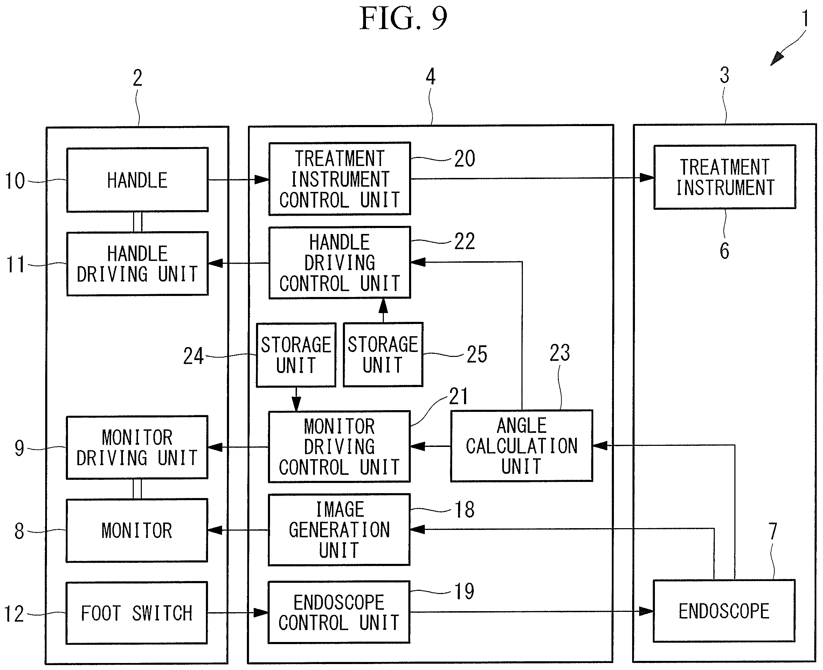

The first function and the second function may respectively be stored in storage units 24 and 25 connected to the monitor driving control unit 21 and the handle driving control unit 22, as illustrated in Fig. 9. The monitor driving control unit 21 and the handle driving control unit 22 may calculate the angles β and γ using the first function and the second function stored in the storage units 24 and 25 based on the angle α calculated by the angle calculation unit 23.

In an example illustrated in Fig. 8, when the angles of the bending portions 7a and 7b in the endoscope 7 change by the operation of the foot switch 12, and the angle α of the optical axis 13a of the camera 13 changes in the angular range of 40° to 50°, the monitor driving control unit 21 and the handle driving control unit 22 perform control so that the angle β of the monitor 8 and the angle γ of the straight line connecting the eyepiece portion 8a and the grip 10a match the angle α.

If the angle α of the optical axis 13a of the camera 13 changes in the angular ranges of 20° to 40° and 50° to 70°, the monitor driving control unit 21 and the handle driving control unit 22 perform control so that the angles β and γ become a value obtained by multiplying the angle α by a proportionality coefficient 0.5. Further, if the angle α of the optical axis 13a of the camera 13 changes in the angular ranges of 0° to 20° and 70° to 90°, the monitor driving control unit 21 and the handle driving control unit 22 perform control so that the angles β and γ become a value obtained by multiplying the angle α by a proportionality coefficient 0.1.

If the angles β and γ are directly proportional to the angle α with the proportionality coefficient 1 over the entire angular range of the angle α, as illustrated in Fig. 3, the grip 10a moves too far from and too close to the body of the doctor D as the angle α comes closer to both ends of the angular range so that a posture of the doctor D becomes cramped. On the other hand, respective movement amounts of the monitor 8 and the grip 10a at both the ends of the angular range of the angle α become small by using the first function and the second function illustrated in Fig. 8. Thus, the posture can be prevented from being too cramped while enabling an intuitive operation of the grip 10a.

While the proportionality coefficient is set to 1, 0.5, and 0.1 in the present embodiment, the present invention is not limited to these. Any proportionality coefficient can be used.

Area division of the angular range is not limited to the aforementioned division, and can optionally be set.

In an area positioned at an extreme end of the angular range, the angles β and γ may not be changed regardless of the change in the angle α.

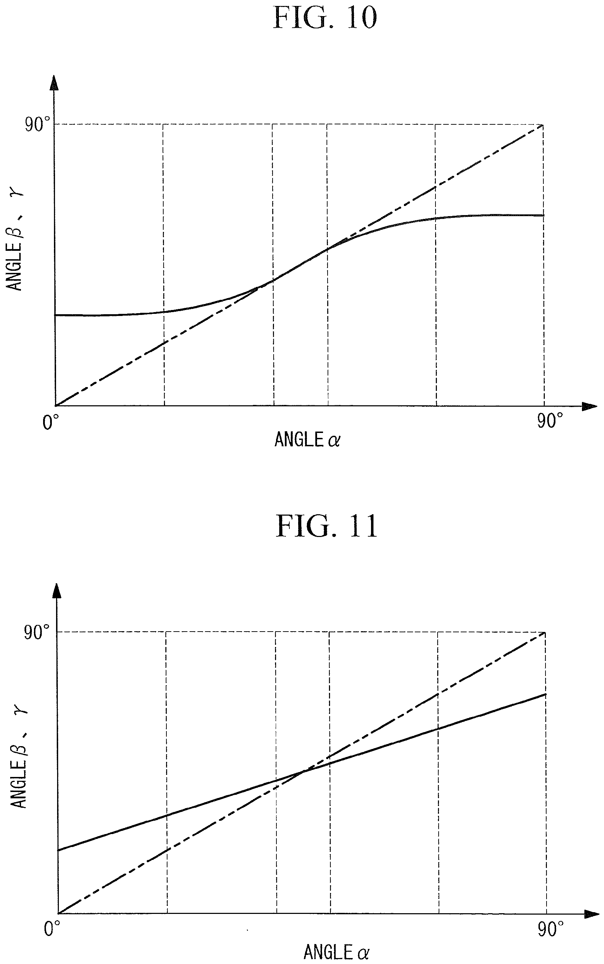

While the proportionality coefficient is discontinuously changed for each area, a function, which continuously changes, may be used without being divided into finite areas, as illustrated in Fig. 10.

As illustrated in Fig. 11, a function, in which angles β and γ are proportional to an angle α with a proportionality coefficient 1 that is less than one, may be used over an entire angular range of the angle α. Thus, the monitor 8 and the grip 10a are operated so that the relative positional relationship between the eyepiece portion 8a and the grip 10a comes closer to the relative positional relationship between the camera 13 and the instrument 6. Thus, intuitivity of the operation of the grip 10a is maintained.

While the first function and the second function are the same function, the present invention is not limited to this. The first and second functions may be different functions. A directly proportional function with a proportionality coefficient 1 may be used as either one of the first and second functions.

While the endoscope 7 of a front-view type in which the optical axis 13a of the camera 13 matches the longitudinal direction of the endoscope 7 is illustrated as the endoscope 7 in the aforementioned embodiment, an endoscope 7 of a sideview type or an oblique-view type may be used. In this case, the endoscope of the respective types differ in a relationship between the bending angles of the bending portions 7a and 7b in the endoscope 7 and the angle α of the optical axis 13a of the camera 13.

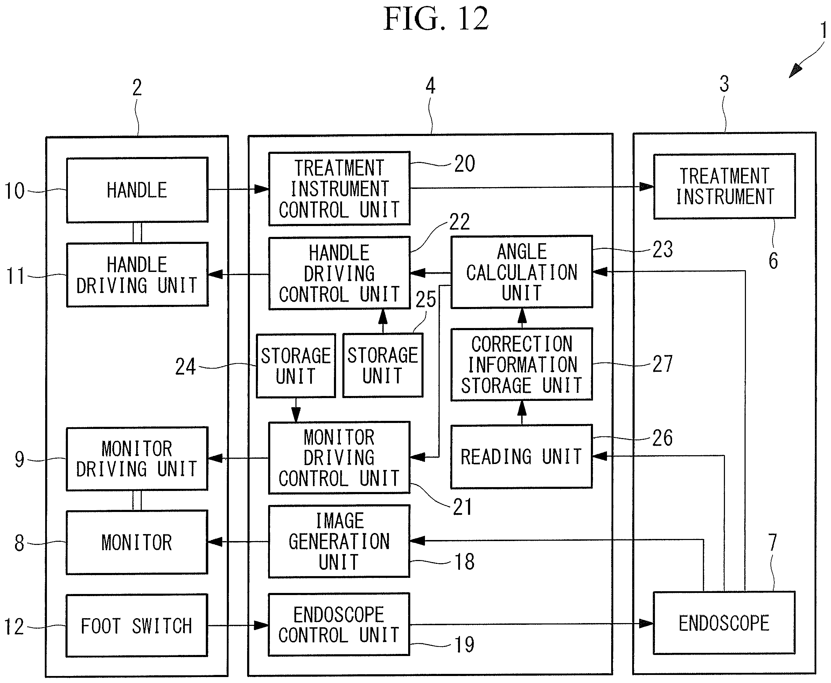

As illustrated in Fig. 12, a controller 4 may include a reading unit 26 that reads type information provided in an endoscope 7 and a correction information storage unit 27 that stores correction information to correspond to the type information, and an angle calculation unit 23 may calculate an angle α using the correction information read out from the correction information storage unit 27 using the type information read by the reading unit 26.

The master device 2 and the slave device 3 may be arranged in proximity to each other, may be electrically connected to each other, or may be arranged remotely from each other, to send and receive a signal by wireless transmission.

### {Reference Signs List}

- A: affected area (subject)
- α: angle (imaging unit angle)
- β: angle (display unit angle)
- γ: angle (operation unit angle)
- 1: manipulator system
- 4: controller (control unit)
- 6: instrument (treatment unit)
- 8: monitor (display unit)
- 9: monitor driving unit (display unit driving unit)
- 10: handle (operation unit)
- 11: handle driving unit (operation unit driving unit)
- 13: camera (imaging unit)
- 24, 25: storage unit (function storage unit)

## Claims

1. A manipulator system (1) comprising:
a treatment unit (6) that treats a subject;
an imaging unit (13) that images the subject and the treatment unit (6);
a display unit (8) that displays an image captured by the imaging unit (13);
an operation unit (10) that operates the treatment unit (6); and **characterized by** further comprising
a display unit driving (9)unit that supports and moves the display unit (8);
an operation unit driving unit (11) that supports and moves the operation unit (10); and
a control unit (4) that controls the display unit driving unit (9) and the operation unit driving unit (11) so that a relative positional relationship between the display unit (8) and the operation unit (10) matches a relative positional relationship between the imaging unit (13) and the treatment unit (6).

2. The manipulator system (1) according to claim 1, further comprising a detection unit that detects a imaging unit angle (α) of the subject by the imaging unit (13),
wherein the control unit (4) controls the display unit driving unit (9) and the operation unit driving unit (11) based on the imaging unit angle (α) detected by the detection unit.

3. The manipulator system (1) according to claim 2, wherein the display unit driving unit (9) changes a display unit angle (β) serving as an angle between a normal to the display unit (8) and a horizontal direction.

4. The manipulator system (1) according to claim 2 or claim 3, wherein the operation unit driving unit (11) changes an operation unit angle (γ) serving as an angle between a straight line connecting the operation unit (10) and the display unit (8) and a horizontal direction.

5. The manipulator system (1) according to claim 3, wherein the control unit (4) controls the display unit driving unit (9) so that the imaging unit angle (α) and the display unit angle (β) match each other.

6. The manipulator system (1) according to claim 4, wherein the control unit (4) controls the operation unit driving unit (11) so that the imaging unit angle (α) and the operation unit angle (γ) match each other.

7. The manipulator system (1) according to claim 3, wherein
the operation unit driving unit (11) changes an operation unit angle (γ) serving as an angle between a straight line connecting the operation unit (10) and the display unit (8) and a horizontal direction, and
the control unit (4) controls the display unit driving unit (9) and the operation unit driving unit (11) so that the imaging unit angle (α), the operation unit angle (γ), and the display unit angle (β) match one another.

8. The manipulator system (1) according to claim 3, further comprising a function storage unit (24, 25) that stores a first function representing a relationship between the imaging unit angle (α) and the display unit angle (β),
wherein the control unit (4) controls the display unit driving (9) unit based on the first function stored in the function storage unit (24, 25).

9. The manipulator system (1) according to claim 8, wherein the first function is a function having a slope decreasing toward both ends of an angular range of the imaging unit angle (α).

10. The manipulator system (1) according to claim 8, wherein the first function is a function in which a change amount of the display unit angle (β) relative to a change amount of the imaging unit angle (α) is one or less over an entire angular range of the imaging unit angle (α).

11. The manipulator system (1) according to claim 4, further comprising a function storage unit (24, 25) that stores a second function representing a relationship between the imaging unit angle (α) and the operation unit angle (γ),
wherein the control unit (4) controls the operation unit driving unit (11) based on the second function stored in the function storage unit (24, 25).

12. The manipulator system (1) according to claim 11, wherein the second function is a function having a slope decreasing toward both ends of an angular range of the imaging unit angle (α).

13. The manipulator system (1) according to claim 11, wherein the second function is a function in which a change amount of the operation unit angle (γ) relative to a change amount of the imaging unit angle (α) is one or less over an entire angular range of the imaging unit angle (α).

## Patentansprüche

1. Manipulatorsystem (1), umfassend:
eine Behandlungseinheit (6), die ein Subjekt behandelt;
eine Abbildungseinheit (13), die das Subjekt und die Behandlungseinheit (6) abbildet;
eine Anzeigeeinheit (8), die ein Bild anzeigt, das von der Abbildungseinheit (13) aufgenommen wurde;
eine Betriebseinheit (10), die die Behandlungseinheit (6) betreibt;
und **dadurch gekennzeichnet, dass** es weiterhin umfasst:
eine Anzeigeeinheitsantriebseinheit (9), die die Anzeigeeinheit (8) trägt und bewegt;
eine Betriebseinheitsantriebseinheit (11), die die Betriebseinheit (10) trägt und bewegt; und
eine Steuereinheit (4), die die Anzeigeeinheitsantriebseinheit (9) und die Betriebseinheitsantriebseinheit (11) so steuert, dass eine relative Positionsbeziehung zwischen der Anzeigeeinheit (8) und der Betriebseinheit (10) mit einer relativen Positionsbeziehung zwischen der Abbildungseinheit (13) und der Behandlungseinheit (6) übereinstimmt.

2. Manipulatorsystem (1) nach Anspruch 1, das weiterhin eine Erfassungseinheit umfasst, die einen Abbildungseinheitswinkel (α) des Subjekts durch die Abbildungseinheit (13) erfasst,
wobei die Steuereinheit (4) die Anzeigeeinheitsantriebseinheit (9) und die Betriebseinheitsantriebseinheit (11) auf Basis des Abbildungseinheitswinkels (α), der von der Erfassungseinheit erfasst wurde, steuert.

3. Manipulatorsystem (1) nach Anspruch 2, wobei die Anzeigeeinheitsantriebseinheit (9) einen Anzeigeeinheitswinkel (β) ändert, der als ein Winkel zwischen einer Normalen zu der Anzeigeeinheit (8) und einer horizontalen Richtung dient.

4. Manipulatorsystem (1) nach Anspruch 2 oder 3, wobei die Betriebseinheitsantriebseinheit (11) einen Betriebseinheitswinkel (γ) ändert, der als ein Winkel zwischen einer geraden Linie, die die Betriebseinheit (10) und die Anzeigeeinheit (8) verbindet, und einer horizontalen Richtung dient.

5. Manipulatorsystem (1) nach Anspruch 3, wobei die Steuereinheit (4) die Anzeigeeinheitsantriebseinheit (9) so steuert, dass der Abbildungseinheitswinkel (α) und der Anzeigeeinheitswinkel (β) übereinstimmen.

6. Manipulatorsystem (1) nach Anspruch 4, wobei die Steuereinheit (4) die Betriebseinheitsantriebseinheit (11) so steuert, dass der Abbildungseinheitswinkel (α) und der Betriebseinheitswinkel (γ) übereinstimmen.

7. Manipulatorsystem (1) nach Anspruch 3, wobei
die Betriebseinheitsantriebseinheit (11) einen Betriebseinheitswinkel (γ) ändert, der als ein Winkel zwischen einer geraden Linie, die die Betriebseinheit (10) und die Anzeigeeinheit (8) verbindet, und einer horizontalen Richtung dient, und
die Steuereinheit (4) die Anzeigeeinheitsantriebseinheit (9) und die Betriebseinheitsantriebseinheit (11) so steuert, dass der Abbildungseinheitswinkel (α), der Betriebseinheitswinkel (γ) und der Anzeigeeinheitswinkel (β) übereinstimmen.

8. Manipulatorsystem (1) nach Anspruch 3, das weiterhin eine Funktionsspeichereinheit (24, 25) umfasst, die eine erste Funktion speichert, die eine Beziehung zwischen dem Abbildungseinheitswinkel (α) und dem Anzeigeeinheitswinkel (β) darstellt,
wobei die Steuereinheit (4) die Anzeigeeinheitsantriebseinheit (9) auf der Basis der ersten Funktion steuert, die in der Funktionsspeichereinheit (24, 25) gespeichert ist.

9. Manipulatorsystem (1) nach Anspruch 8, wobei die erste Funktion eine Funktion mit einer Neigung ist, die zu beiden Enden eines Winkelbereichs des Abbildungseinheitswinkels (α) hin abnimmt.

10. Manipulatorsystem (1) nach Anspruch 8, wobei die erste Funktion eine Funktion ist, in der ein Veränderungsbetrag des Anzeigeeinheitswinkels (β) in Bezug auf einen Veränderungsumfang des Abbildungseinheitswinkels (α) eins oder weniger gegenüber einem Gesamtwinkelbereich des Abbildungseinheitswinkels (α) ist.

11. Manipulatorsystem (1) nach Anspruch 4, das weiterhin eine Funktionsspeichereinheit (24, 25) umfasst, die eine zweite Funktion speichert, die eine Beziehung zwischen dem Abbildungseinheitswinkel (α) und dem Betriebseinheitswinkel (γ) darstellt,
wobei die Steuereinheit (4) die Betriebseinheitsantriebseinheit (11) auf der Basis der zweiten Funktion steuert, die in der Funktionsspeichereinheit (24, 25) gespeichert ist.

12. Manipulatorsystem (1) nach Anspruch 11, wobei die zweite Funktion eine Funktion mit einer Neigung ist, die zu beiden Enden eines Winkelbereichs des Abbildungseinheitswinkels (α) hin abnimmt.

13. Manipulatorsystem (1) nach Anspruch 11, wobei die zweite Funktion eine Funktion ist, in der ein Veränderungsbetrag des Betriebseinheitswinkels (γ) in Bezug auf einen Veränderungsbetrag des Abbildungseinheitswinkels (α) eins oder weniger über einen Gesamtwinkelbereich des Abbildungseinheitswinkels (α) ist.

## Revendications

1. Système de manipulateur (1) comprenant :
une unité de traitement (6) qui traite un sujet ;
une unité d'imagerie (13) qui réalise une imagerie du sujet et de l'unité de traitement (6) ;
une unité d'affichage (8) qui affiche une image capturée par l'unité d'imagerie (13) ;
une unité d'actionnement (10) qui actionne l'unité de traitement (6) ; et
**caractérisé par le fait qu'**il comprend en outre
une unité (9) d'entraînement d'unité d'affichage qui supporte et déplace l'unité d'affichage (8) ;
une unité (11) d'entraînement d'unité d'actionnement qui supporte et déplace l'unité d'actionnement (10) ; et
une unité de commande (4) qui commande l'unité (9) d'entraînement d'unité d'affichage et l'unité (11) d'entraînement d'unité d'actionnement de telle sorte qu'une relation de position relative entre l'unité d'affichage (8) et l'unité d'actionnement (10) correspond à une relation de position relative entre l'unité d'imagerie (13) et l'unité de traitement (6).

2. Système de manipulateur (1) selon la revendication 1, comprenant en outre une unité de détection qui détecte un angle d'unité d'imagerie (α) du sujet par l'unité d'imagerie (13),
l'unité de commande (4) commandant l'unité (9) d'entraînement d'unité d'affichage et l'unité (11) d'entraînement d'unité d'actionnement sur la base de l'angle d'unité d'imagerie (α) détecté par l'unité de détection.

3. Système de manipulateur (1) selon la revendication 2, dans lequel l'unité (9) d'entraînement d'unité d'affichage change un angle d'unité d'affichage (β) servant d'angle entre une normale à l'unité d'affichage (8) et une direction horizontale.

4. Système de manipulateur (1) selon la revendication 2 ou la revendication 3, dans lequel l'unité (11) d'entraînement d'unité d'actionnement change un angle d'unité d'actionnement (γ) servant d'angle entre une ligne droite reliant l'unité d'actionnement (10) et l'unité d'affichage (8) et une direction horizontale.

5. Système de manipulateur (1) selon la revendication 3, dans lequel l'unité de commande (4) commande l'unité (9) d'entraînement d'unité d'affichage de telle sorte que l'angle d'unité d'imagerie (α) et l'angle d'unité d'affichage (β) sont mis en correspondance l'un avec l'autre.

6. Système de manipulateur (1) selon la revendication 4, dans lequel l'unité de commande (4) commande l'unité (11) d'entraînement d'unité d'actionnement de telle sorte que l'angle d'unité d'imagerie (α) et l'angle d'unité d'actionnement (γ) sont mis en correspondance l'un avec l'autre.

7. Système de manipulateur (1) selon la revendication 3, dans lequel
l'unité (11) d'entraînement d'unité d'actionnement change un angle d'unité d'actionnement (γ) servant d'angle entre une ligne droite reliant l'unité d'actionnement (10) et l'unité d'affichage (8) et une direction horizontale, et l'unité de commande (4) commande l'unité (9) d'entraînement d'unité d'affichage et l'unité (11) d'entraînement d'unité d'actionnement de telle sorte que l'angle d'unité d'imagerie (α), l'angle d'unité d'actionnement (γ) et l'angle d'unité d'affichage (β) sont mis en correspondance les uns avec les autres.

8. Système de manipulateur (1) selon la revendication 3, comprenant en outre une unité de stockage de fonction (24, 25) qui stocke une première fonction représentant une relation entre l'angle d'unité d'imagerie (α) et l'angle d'unité d'affichage (β),
l'unité de commande (4) commandant l'unité (9) d'entraînement d'unité d'affichage sur la base de la première fonction stockée dans l'unité de stockage de fonction (24, 25).

9. Système de manipulateur (1) selon la revendication 8, dans lequel la première fonction est une fonction ayant une pente diminuant vers les deux limites d'une plage angulaire de l'angle d'unité d'imagerie (α).

10. Système de manipulateur (1) selon la revendication 8, dans lequel la première fonction est une fonction dans laquelle une grandeur de variation de l'angle d'unité d'affichage (β) par rapport à une grandeur de variation de l'angle d'unité d'imagerie (α) est un ou moins sur toute une plage angulaire de l'angle d'unité d'imagerie (α).

11. Système de manipulateur (1) selon la revendication 4, comprenant en outre une unité de stockage de fonction (24, 25) qui stocke une seconde fonction représentant une relation entre l'angle d'unité d'imagerie (α) et l'angle d'unité d'actionnement (γ),
l'unité de commande (4) commandant l'unité (11) d'entraînement d'unité d'actionnement sur la base de la seconde fonction stockée dans l'unité de stockage de fonction (24, 25).

12. Système de manipulateur (1) selon la revendication 11, dans lequel la seconde fonction est une fonction ayant une pente diminuant vers les deux limites d'une plage angulaire de l'angle d'unité d'imagerie (α).

13. Système de manipulateur (1) selon la revendication 11, dans lequel la seconde fonction est une fonction dans laquelle une grandeur de variation de l'angle d'unité d'actionnement (γ) par rapport à une grandeur de variation de l'angle d'unité d'imagerie (α) est un ou moins sur toute une plage angulaire de l'angle d'unité d'imagerie (α).
